(19) Europäisches Patentamt
European Patent Office
Office européen des brevets

(11) Publication number: **0 022 607**
**B1**

(12) # EUROPEAN PATENT SPECIFICATION

(45) Date of publication of patent specification: **12.01.83**

(51) Int. Cl.³: **C 07 C 21/14**, C 07 C 21/04, C 07 C 17/34

(21) Application number: **80301417.4**

(22) Date of filing: **30.04.80**

(54) **Process for the preparation of halogenated hydrocarbons.**

(30) Priority: **13.07.79 GB 7924524**

(43) Date of publication of application:
**21.01.81 Bulletin 81/3**

(45) Publication of the grant of the patent:
**12.01.83 Bulletin 83/2**

(84) Designated Contracting States:
**CH DE FR GB IT LI NL**

(56) References cited:
**US - A - 3 116 337**
**US - A - 4 070 404**

(73) Proprietor: **IMPERIAL CHEMICAL INDUSTRIES PLC**
**Imperial Chemical House Millbank**
**London SW1P 3JF (GB)**

(72) Inventor: **Crosby, John**
**12 Oakwood Court**
**Bowdon, Altrincham Cheshire WA14 3DJ (GB)**
Inventor: **Milner, David John**
**18 Eight Acre**
**Whitefield, Manchester M25 7LW (GB)**
Inventor: **Terry, Bernard William Hugh**
**188 Blackley New Road**
**Blackley Manchester M9 3FQ (GB)**

(74) Representative: **Clark, Peter Frederick et al,**
**Imperial Chemical Industries PLC Legal Department, Patents Thames House North Millbank**
**London SW1P 4QG (GB)**

**EP 0 022 607 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for the preparation of halogenated hydrocarbons

This invention relates to a process for the preparation of halogenated hydrocarbons which are useful as insecticide intermediates.

Compounds having the formula:

$$X_3C—CH_2—CH{=}C(CH_3)_2 \qquad (I)$$

wherein each X is chlorine or bromine and may be the same or different, are valuable intermediates in the synthesis of pyrethroid insecticides. Thus, these intermediates may be reacted with alkyl diazoacetates $N_2CH.COOR$, in which R is an alkyl group, to give cyclopropane derivatives having the formula:

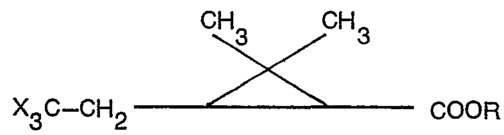

(II)

This reaction is carried out in the presence of a catalyst which may be, for example, copper powder, copper bronze, a rhodium (II) carboxylate such as rhodium (II) pivalate, copper complexes of Schiff bases as described in United Kingdom Patent Specification No. 1455189 and certain chiral copper (II) complexes with Schiff base ligants derived from amino-sugars having a hydroxyl group on the carbon atom adjacent that bearing the amino group. The process and certain catalysts for use therein are the subject of copending patent applications or even date.

Compounds of formula (II) may be dehydrohalogenated, for example, by reaction with an alkali metal carbonate in a polar aprotic solvent, to give compounds of formula:

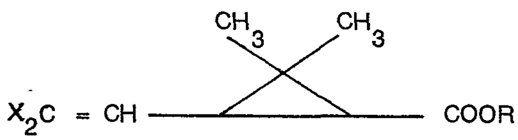

(III)

and these latter compounds may be converted by conventional methods of organic chemistry, e.g. via the corresponding carboxylic acid chloride or by transesterification methods, into compounds of formula:

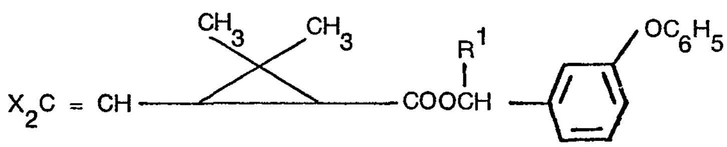

(IV)

in which $R^1$ is H, CN or —C≡CH, which compounds are known insecticides.

The route to compounds of formula (III) indicated above, i.e. reaction of the monoene of formula (I) with diazoacetic ester to give the compound of formula (II) followed by dehydrohalogenation, has advantages over the more direct route of addition of the diazoacetic ester to a diene of formula

$$X_2C{=}CH—CH{=}C(CH_3)_2$$

in that the diazoacetic ester/monoene addition favours the formation of the *cis* isomer and, especially when suitable chiral catalysts are used, the *cis* IR isomer of (II); the insecticidal esters of formula (V) which are obtained from compounds of formula (II) have the maximum insecticidal potency in the *cis* IR configuration.

The compound of formula (I) in which each X is chlorine is known, but there is no satisfactory method for its preparation.

US Patent Specification No. 4070404 discloses the addition of carbon tetrachloride and of bromotrichloromethane to 3-methylbut-1-ene in the presence of a radical initiator to give 1,1,1,3-tetrachloro-4-methylpentane and 3-bromo-1,1,1-trichloro-4-methylpentane respectively. Dehydrobromination of the latter compound by means of potassium hydroxide in ethanol at 0°C is stated to give 58.5% of 1,1,1-trichloro-4-methylpent-2-ene and only 8.1% of the required 1,1,1-trichloro-4-methylpent-3-ene. Dehydrochlorination of 1,1,1,3-tetrachloro-4-methylpentane under similar conditions is stated to give 45.2% of 1,1,1-trichloro-4-methylpent-2-ene and 44.8% of 1,1,1-trichloro-4-methylpent-3-ene.

However, on repeating the latter preparation (5°C for 16 hours) it is found that no 1,1,1-trichloro-4-methylpent-3-ene appears to be formed, and that the product has properties consistent with the formation of 1,1,3-trichloro-4-methylpent-1-ene and 1,1,1-trichloro-4-methylpent-2-ene in approximately equimolar proportions. This conclusion is supported by the mass spectrum of the mixture and by its NMR spectrum, which showed $\delta$ 6.0, m, 7.0H, vinylic; $\delta$ 4.5, q, 2.0H, allylic; $\delta$ 2.4, q, 2.0H, allylic; $\delta$ 1.0, m, 33H, aliphatic.

Authentic 1,1,1-trichloro-4-methylpent-3-ene had $\delta$ 1.75, d, 6.5H; $\delta$ 3.35, d, 2.0H; $\delta$ 5.38, t, 0.9H, and clearly this compound is not a significant component of the mixture.

Similarly, Farkas, Kourim and Sorm, *Coll. Czech. Chem. Comm., 24,* 2230 (1959), prepared 1,1,1,3-tetrachloro-4-methylpentane in 38% yield by reaction of carbon tetrachloride with 3-methylbut-1-ene in the presence of benzoyl peroxide. Dehydrochlorination using 1 mol of ethanolic potassium hydroxide at 0°C gave what was believed to be 1,1,1-trichloro-4-methylpent-2-ene. Using 2 mols of potassium hydroxide at 20°C there was obtained a mixture comprising 90% of 1,1,1-trichloro-4-methylpent-2-ene (or possibly 1,1,3-trichloro-4-methylpent-1-ene) and 10% of 1,1-dichloro-3-ethoxy-4-methylpent-1-ene. Apparently in neither case was 1,1,1-trichloro-4-methylpent-3-ene obtained.

It has now been found that 1,1,1-trihalo-4-methylpent-3-enes may be obtained as the major component by the dehydrohalogenation of a 1,1,1,3-tetrahalo-4-methylpentane under specific conditions.

According to the present invention there is provided a process for the manufacture of compounds having the formula:

$$X_3C{-}CH_2{-}CH{=}C(CH_3)_2$$

wherein each X independently represents chlorine or bromine, which comprises heating a compound having the formula:

$$X_3C{-}CH_2{-}\underset{\displaystyle}{\overset{\displaystyle Hal}{\overset{|}{C}H}}{-}CH(CH_3)_2$$

wherein Hal represents chlorine or bromine, with an alkali metal halide in a polar aprotic solvent.

Examples of the alkali metal halides which may be used are the fluorides, chlorides, bromides or iodides of lithium, sodium and potassium. A preferred alkali metal halide is lithium bromide.

Examples of the polar aprotic solvents which may be used are dimethylacetamide, diethylformamide, hexamethylphosphoramide, and dimethylformamide.

The amount of polar aprotic solvent which is used may be from 1 to 200 moles, preferably 2 to 20 moles, per mole of 1,1,1,3-tetrahalo-4-methylpentane.

The amount of alkali metal halide which is used may be from 0.01 to 10.0 moles, preferably 0.1 to 2.0 moles per mole of 1,1,1,3-tetrahalo-4-methylpentane.

The reaction may be carried out at a temperature from 50 to 300°C, conveniently from 70 to 150°C, and may require a reaction time of from a few minutes to a few days depending on the temperature used.

When the starting material is 3-bromo-1,1,1-trichloro-4-methylpentane, the reaction is conveniently carried out by simply heating the reactants together for several hours at about 80°C.

When 1,1,1,3-tetrachloro-4-methylpentane is used as the starting material a preferred procedure is to carry out the reaction at about 100°C and slowly to distil the products out of the reaction mixture under reduced pressure as they are formed. Further quantities of alkali metal halide and polar aprotic solvent may be added to the residue from the distillation and the procedure repeated one or more times in order to convert more of the starting material into the desired product.

The reaction product may be isolated from the reaction mixture by conventional means. For example, the mixture may be diluted with a water-immiscible solvent such as dichloromethane, washed with water to remove water-soluble salts and other materials, dried and fractionally distilled. Another convenient procedure for product isolation is by steam distillation from the reaction mixture.

The invention is illustrated by the following Examples in which percentages are by weight unless otherwise stated.

Example 1

3-Bromo-1,1,1-trichloro-4-methylpentane (46.6 g; prepared as described below) and anhydrous lithium bromide (9.3 g) were placed in a 1-litre flask fitted with a condenser. Dimethylformamide (185 ml) was added, and the mixture was heated in an oil-bath at 78—80°C for 5 hours. After cooling the reaction mixture, methylene chloride (200 ml) was added and the solution was washed with water 5 x 200 ml) and dried over anhydrous magnesium sulphate. Fractional distillation afforded a fraction (22.4 g) boiling at 60 to 70°/15 mm Hg.

Examination of this fraction by gas-liquid chromatography (GLC) (1.52 m 10% E301 Column at

150°C) showed predominantly one component together with a small amount (ca. 5%) of lower boiling product. 'Hnmr spectroscopy of the major fraction showed $\delta$1.75 (6.5 H, d), $\delta$3.35 (2.0 H, d). $\delta$5.38 (0.9 H, t) which is consistent with the structure

$$Cl_3C—CH_2—CH=C(CH_3)_2.$$

The 3-bromo-1,1,1-trichloro-4-methylpentane used in the above preparation was obtained as follows:—

Cuprous chloride (0.2 g) was dissolved in monoethanolamine (6.1 g) in a glass autoclave liner. Tertiary butanol (150 ml) and bromotrichloromethane (49.6 g) were then added and the mixture was cooled in an ice-bath. 3-Methylbut-1-ene (14.0 g) was added and the liner was sealed in an autoclave. The mixture was heated under autogeneous pressure for 24 hours at 100°C.

The autoclave was allowed to cool and the reaction mixture was distilled. Low boiling material was removed at atmospheric pressure at a distillation head temperature up to 82°C. Fractional distillation of the residue gave 3-bromo-1,1,1-trichloro-4-methylpentane (27.6 g, 51% yield based on 3-methylbut-1-ene), b.p. 99—104°C/16 mm Hg.

Example 2

1,1,1,3-Tetrachloro-4-methylpentane (86.9 g; 0.388 mol) was placed in a 1 litre flask together with lithium bromide (17.4 g) and dry dimethylformamide (500 ml). The mixture was heated to 100—105°C and the pressure of the system was reduced so that the dimethylformamide and products could be removed by distillation through a 1 m column of Fenske helices. The distillate was collected using a Perkin distillation triangle at a reflux ratio of 8:1. At the beginning of the reaction, a pressure of 150 mm Hg was needed for the distillation. This pressure required periodic adjustment to maintain reflux of the reaction mixture. When 450 ml of distillate had been collected the pressure required for distillation was 70 mm Hg.

The procedure was repeated twice more by adding successively two 500 ml portions of dimethylformamide each containing lithium bromide (26.1 g). The total time required for the reaction and distillation was 16 hours. The distillate (1.5 l) was mixed with methylene chloride (1.5 l) and the mixture was washed with water (7 x 1.5 l). The organic layer was dried over anhydrous magnesium sulphate and fractionally distilled. There was obtained a mixture (30.7 g) which was shown by GLC analysis to contain 1,1,1-trichloro-4-methylpent-3-ene (25.6 g; 0.137 mol) and 1,1-dichloro-4-methylpenta-1,3-diene (5.1 g; 0.033 mol). The mass spectra of the GLC components are identical with authentic samples of these two compounds. A residue contained 1,1,1,3-tetrachloro-4-methylpentane (51.1 g; 0.218 mol).

In this reaction 0.170 mol of starting material had been consumed and 0.170 mol of mixed products formed, in a mol. ratio of 80% 1,1,1-trichloro-4-methylpent-3-ene to 20% 1,1-dichloro-4-methylpenta-1,3-diene.

## Claims

1. A process for the preparation of halogenated hydrocarbons having the formula:

$$X_3C—CH_2—CH=C(CH_3)_2$$

wherein each X independently represents chlorine or bromine characterised in that a 1,1,1,3-tetrahalo-4-methylpentane having the formula:

$$X_3C—CH_2—\overset{\displaystyle Hal}{\overset{|}{C}H}—CH(CH_3)_2$$

in Hal represents chlorine or bromine, is heated with an alkali metal halide in a polar aprotic solvent.

2. A process as claimed in claim 1 characterised in that the alkali metal halide is lithium bromide.

3. A process as claimed in claim 1 or claim 2 characterised in that the polar aprotic solvent is dimethyl formamide.

4. A process as claimed in any one of claims 1 to 3 characterised in that the amount of polar aprotic solvent which is used is from 1 to 200 moles per mole of 1,1,1,3-tetrahalo-4-methylpentane.

5. A process as claimed in claim 4 characterised in that the amount of polar aprotic solvent which is used is from 2 to 20 moles per mole of 1,1,1,3-tetrahalo-4-methylpentane.

6. A process as claimed in any one of claims 1 to 5 characterised in that the amount of alkali metal halide which is used is from 0.01 to 10.0 moles per mole of 1,1,1,3-tetrahalo-4-methylpentane.

7. A process as claimed in claim 6 characterised in that the amount of alkali metal halide which is used is from 0.1 to 2.0 moles per mole of 1,1,1,3-tetrahalo-4-methylpentane.

8. A process as claimed in any one of claims 1 to 7 characterised in that the reaction is carried out at a temperature from 50 to 300°C.

9. A process as claimed in claim 8 characterised in that the reaction is carried out at a temperature from 70 to 150°C.

**Revendications**

1. Procédé de préparation d'hydrocarbures halogénés de formule:

$$X_3C—CH_2—CH{=}C(CH_3)_2$$

dans laquelle chaque X représente, indépendamment, du chlore ou du brome, caractérisé en ce qu'un 1,1,1,3-tétrahalogéno-4-méthylpentane de formule:

$$X_3C—CH_2—\underset{}{\overset{\displaystyle Hal\atop|}{C}}H—CH(CH_3)_2$$

dans laquelle Hal représente le chlore ou le brome, est chauffé avec un halogénure de métal alcalin dans un solvant aprotique polaire.

2. Procédé suivant la revendication 1, caractérisé en ce que l'halogénure de métal alcalin est le bromure de lithium.

3. Procédé suivant la revendication 1 ou la revendication 2, caractérisé en ce que le solvant aprotique polaire est est le diméthylformamide.

4. Procédé suivant l'une quelconque des revendications 1 à 3, caractérisé en ce que la quantité de solvant aprotique polaire qui est utilisée est de 1 à 200 moles par mole de 1,1,1,3-tétrahalogéno-4-méthylpentane.

5. Procédé suivant la revendication 4, caractérisé en ce que la quantité de solvant aprotique polaire qui est utilisée est de 2 à 20 moles par mole de 1,1,1,3-tétrahalogéno-4-méthylpentane.

6. Procédé suivant l'une quelconque des revendications 1 à 5, caractérisé en ce que la quantité d'halogénure de métal alcalin qui est utilisée est de 0,01 à 10,0 moles par mole de 1,1,1,3-tétrahalogéno-4-méthylpentane.

7. Procédé suivant la revendication 6, caractérisé en ce que la quantité d'halogénure de métal alcalin qui est utilisée est de 0,1 à 2,0 moles par mole de 1,1,1,3-tétrahalogéno-4-méthylpentane.

8. Procédé suivant l'une quelconque des revendications 1 à 7, caractérisé en ce que la réaction est conduite à une température de 50 à 300°C.

9. Procédé suivant la revendication 8, caractérisé en ce que la réaction est conduite à une température de 70 à 150°C.

**Patentansprüche**

1. Verfahren zur Herstellung von halogenierten Kohlenwasserstoffen der Formel:

$$X_3C—CH_2—CH{=}C(CH_3)_2$$

worin jedes X unabhängig voneinander für Chlor oder Brom steht, dadurch gekennzeichnet, daß man ein 1,1,1,3-Tetrahalogen-4-methylpentan der Formel:

$$X_3C—CH_2—\overset{\displaystyle Hal\atop|}{C}H—CH(CH_3)_2$$

worin Hal für Chlor oder Brom steht, mit einem Alkalimetallhalogenid in einem polaren aprotischen Lösungsmittel erhitzt.

2. Verfahren nach Anspruch 1, dadurch gekennzeichnet, daß das Alkalimetallhalogenid Lithiumbromid ist.

3. Verfahren nach Anspruch 1 oder 2, dadurch gekennzeichnet, daß das polare aprotische Lösungsmittel Dimethylformamid ist.

4. Verfahren nach einem der Ansprüche 1 bis 3, dadurch gekennzeichnet, daß die verwendete Menge des polaren aprotischen Lösungsmittels 1 bis 200 Mol je Mol 1,1,1,3-Tetrahalogen-4-methylpentan beträgt.

5. Verfahren nach Anspruch 4, dadurch gekennzeichnet, daß die verwendete Menge des polaren aprotischen Lösungsmittels 2 bis 20 Mol je Mol 1,1,1,3-Tetrahalogen-4-methylpentan beträgt.

6. Verfahren nach einem der Ansprüche 1 bis 5, dadurch gekennzeichnet, daß die verwendete

Menge des Alkalimetallhalogenids 0,01 bis 10,0 Mol je Mol 1,1,1,3-Tetrahalogen-4-methylpentan beträgt.

7. Verfahren nach Anspruch 6, dadurch gekennzeichnet, daß die verwendete Menge des Alkalimetallhalogenids 0,1 bis 2,0 Mol je Mol 1,1,1,3-Tetrahalogen-4-methylpentan beträgt.

8. Verfahren nach einem der Ansprüche 1 bis 7, dadurch gekennzeichnet, daß die Reaktion bei einer Temperatur von 50 bis 300°C ausgeführt wird.

9. Verfahren nach Anspruch 8, dadurch gekennzeichnet, daß die Reaktion bei einer Temperatur von 70 bis 150°C ausgeführt wird.